# EUROPEAN PATENT SPECIFICATION

(11) **EP 2 707 640 B1**
(45) Date of publication and mention of the grant of the patent: **03.01.2018**
(21) Application number: 12781675.9
(22) Date of filing: 09.05.2012
(51) Int. Cl.: F16L 37/28

(54) **VALVE FOR REGULATING THE FLOW OF A LIQUID**
VENTIL ZUR REGELUNG DES DURCHFLUSSES EINER FLÜSSIGKEIT
ROBINET DE RÉGULATION DE L'ÉCOULEMENT D'UN LIQUIDE

(30) Priority: 11.05.2011 US 201113105640; 22.12.2011 US 201113334605
(43) Date of publication of application: 19.03.2014
(73) Proprietor: Bioflo, LLC, Sanford, FL 32773 (US)
(72) Inventor: SPOLSKI, Kevin, J., Sanford, FL 32773 (US)
(74) Representative: Sturm, Christoph
(86) International application number: PCT/US2012/037020
(87) International publication number: WO 2012/154781

(56) References cited:
- WO-A1-2004/098686
- WO-A1-2005/004974
- US-A- 2 434 167
- US-A- 2 434 167
- US-A- 2 792 194
- US-A- 4 103 686
- US-A- 4 160 383
- US-A- 4 458 719
- US-A- 5 203 365
- US-A- 5 330 155
- US-B1- 6 488 047

## Description

### BACKGROUND OF THE INVENTION

This application claims priority of U.S. Patent Application serial number 13/334,609, filed on December 22, 2011 and U.S Application serial number 13/105,640, filed on May 11, 2011, which is a continuation-in-part application thereof.

### FIELD OF THE INVENTION

A new design for a valve that regulates the flow of a liquid has the ability to passively regulate the flow of liquid and allows the valve housing to be disconnected, allowing movement of the person to whom the valve is connected away from a collection bag.

Many people, at home, in a hospital, and in third-party care facilities require the use of an in-dwelling bladder catheter because of a medical condition. However, being constantly attached to a bladder bag that holds the liquid (urine) is inconvenient, and potentially and unnecessarily limits the person's movement and living conditions.

A new valve allows for the cyclical emptying of the bladder and allows the patient to be disconnected from the collection bag. Document WO 2004/098686 A1 discloses a bladder cycler for use with catheters comprising a magnetic valve. Document WO 2005/004974 A1 discloses a coupling device with a valve.

### SUMMARY OF THE INVENTION

The present invention is directed to a valve for regulating the flow of a liquid therethrough that includes a first housing having an inlet and an outlet, the first housing having a first magnet housing disposed therein, the first magnet housing having a base, the base having a first side and a second side and at least two extensions extending from the first side, the at least two extensions engaging an inside surface of the first housing and retaining a magnet between the first magnet housing and the first housing, a second housing removably attachable to the first housing, the second housing having an opening extending therethrough, and a sealing member disposed in the first housing adjacent the outlet, the sealing member sealing the outlet in the first housing when the second housing is disengaged from the first housing.

In some embodiments, a sampling port is disposed in the first housing and wherein the at least two extensions are disposed around an opening in the base and have an angle between them, the angle being 180 degrees or less, the at least two extensions being disposed in the first housing such the sampling port bisects the angle between the at least two extensions when the first magnet housing is disposed within the first housing.

In another aspect, the invention is directed to a valve for regulating the flow of a liquid therethrough that includes a first housing having an inlet and an outlet, the first housing having a first magnet housing disposed therein, the first magnet housing having a base, the base having a first side and a second side and at least two extensions extending from the first side, the at least two extensions engaging an inside surface of the first housing and retaining a first magnet between the first magnet housing and the first housing, a second housing removably attachable to the first housing, the second housing having an opening extending therethrough, a second magnet disposed in the first housing, the second magnet movable relative to the first magnet, and a sealing member disposed in the first housing adjacent the outlet, the sealing member sealing the outlet in the first housing when the second housing is disengaged from the first housing.

Additional features and advantages of the invention will be set forth in the detailed description which follows, and in part will be readily apparent to those skilled in the art from that description or recognized by practicing the invention as described herein, including the detailed description which follows, the claims, as well as the appended drawings.

It is to be understood that both the foregoing general description and the following detailed description of the present embodiments of the invention, and are intended to provide an overview or framework for understanding the nature and character of the invention as it is claimed. The accompanying drawings are included to provide a further understanding of the invention, and are incorporated into and constitute a part of this specification. The drawings illustrate various embodiments of the invention, and together with the description serve to explain the principles and operations of the invention.

### BRIEF DESCRIPTION OF THE DRAWINGS

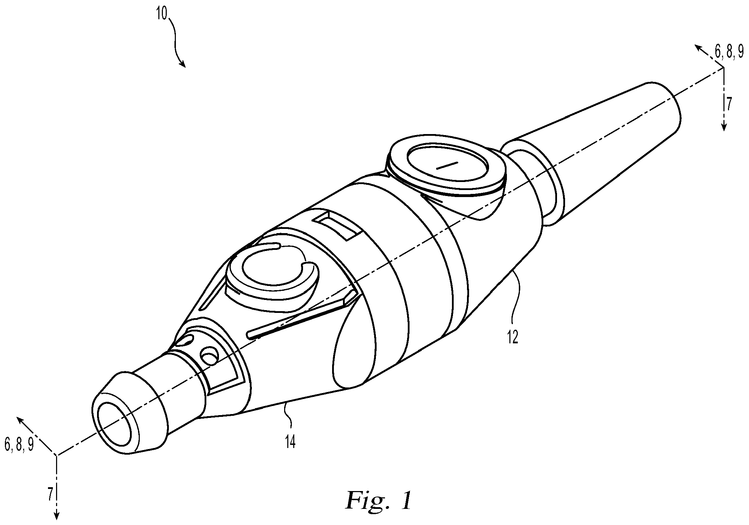

Fig. 1 is a front perspective view of one embodiment of a valve for regulating the flow of a liquid according to the present invention;

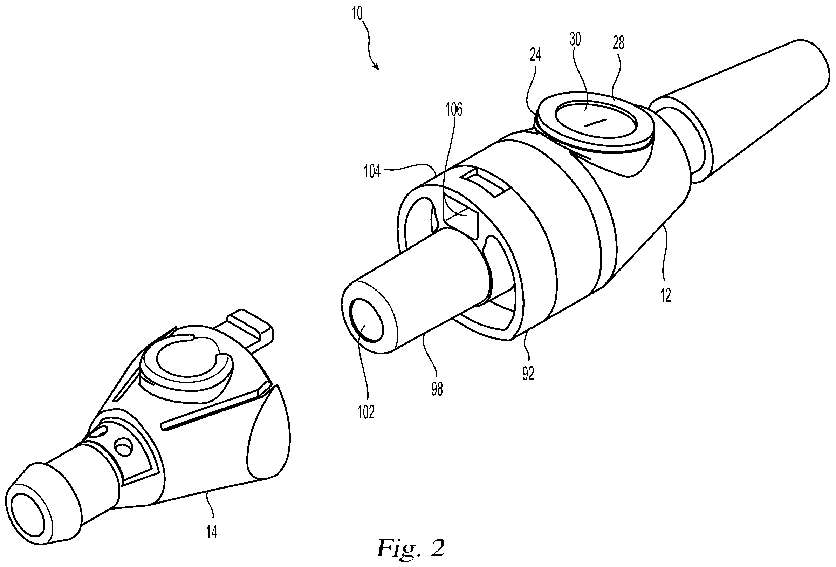

Fig. 2 is a front perspective view of the housings of the valve in Fig. 1 separated from one another;

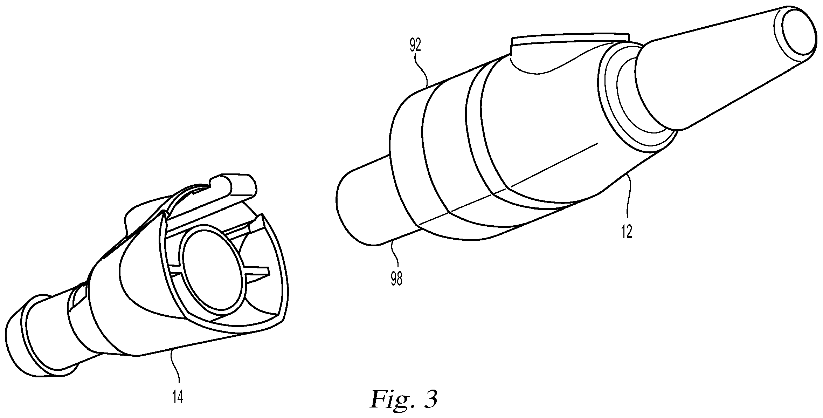

Fig. 3 is a rear perspective view of the housings of the valve in Fig. 1 separated from one another;
Fig. 4 is an exploded perspective view of the valve of Fig 1;
Fig. 5 is a cross sectional view from the left side of the valve in Fig. 4;
Fig. 6 is a cross section view of the valve along the line 6-6- in Fig. 1 with one magnet in a first position and sealing the valve;
Fig. 7 is a cross section view of the valve along the line 7-7 in Fig. 1;
Fig. 8 is a cross section view of the valve along the line 8-8 in Fig. 1 with the magnet in a second position and allowing liquid to flow therethrough ;
Fig. 9 is a cross section view of the valve along the line 9-9 in Fig. 2;
Fig. 10 is a perspective view of the valve connected to tubing on one end and a collection bag on the other end;
Fig. 11 is an exploded perspective view of another embodiment of a valve for regulating the flow of a liquid according to the present invention;
Fig. 12 is a cross sectional view of the left side of the valve in Fig. 11;
Fig. 13 is a cross sectional view of the left side of the valve in Fig. 11 in an assembled state;
Fig. 14 is a cross sectional view from the top of the valve in Fig. 11 in an assembled state;
Fig. 15 is a perspective view of the first magnet holder used in the valve in Fig. 11;
Fig. 16 is a cross sectional view of the left side of the valve in Fig. 11 with the housings separated;
Fig. 17 is a cross sectional view through the first magnet housing toward the inlet in the valve in Fig. 11; and
Fig. 18 is a cross sectional view of a portion of the first housing of the valve in Fig. 11.

### DETAILED DESCRIPTION OF THE PREFERRED EMBODIMENTS

Reference will now be made in detail to the present preferred embodiment(s) of the invention, examples of which are illustrated in the accompanying drawings. Whenever possible, the same reference numerals will be used throughout the drawings to refer to the same or like parts.

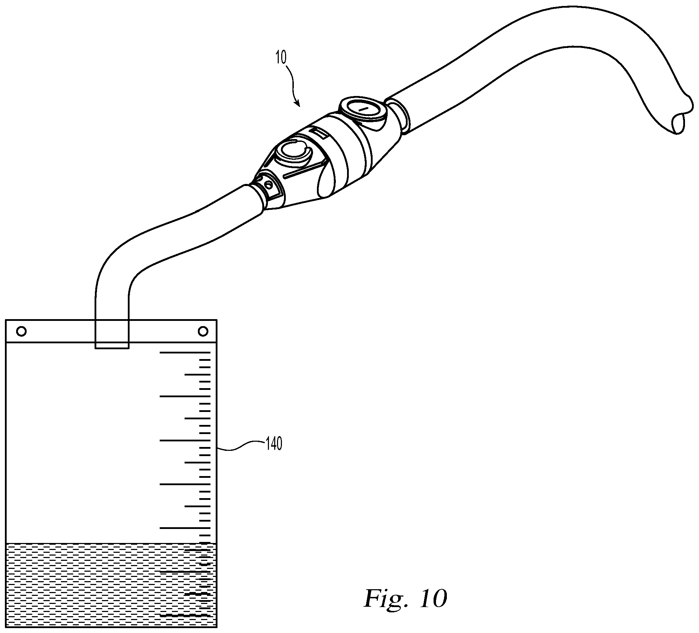

One embodiment of the present invention illustrated in the figures is directed is a valve **10** for regulating the flow of a liquid. The valve **10** has a first housing **12** and a second housing **14** that is removably attachable to the first housing **12.** The first housing **12** has a catheter connector **16** having a proximal end **18** and a distal end **20,** the proximal end **18** of the catheter connector **16** has an inlet **22** that is configured to engage a tubing (see, e.g., Fig. 10). The inlet **22,** as illustrated, has a generally smooth outer surface that increases in diameter from the end of the inlet **22** toward the remaining portion of the catheter connector **16.** The inlet **22** may have any other configuration that allows for connection to tubing and still fall within the scope of the present invention. The first and second housings **12,14** are preferably made from a K-Resin SBC material.

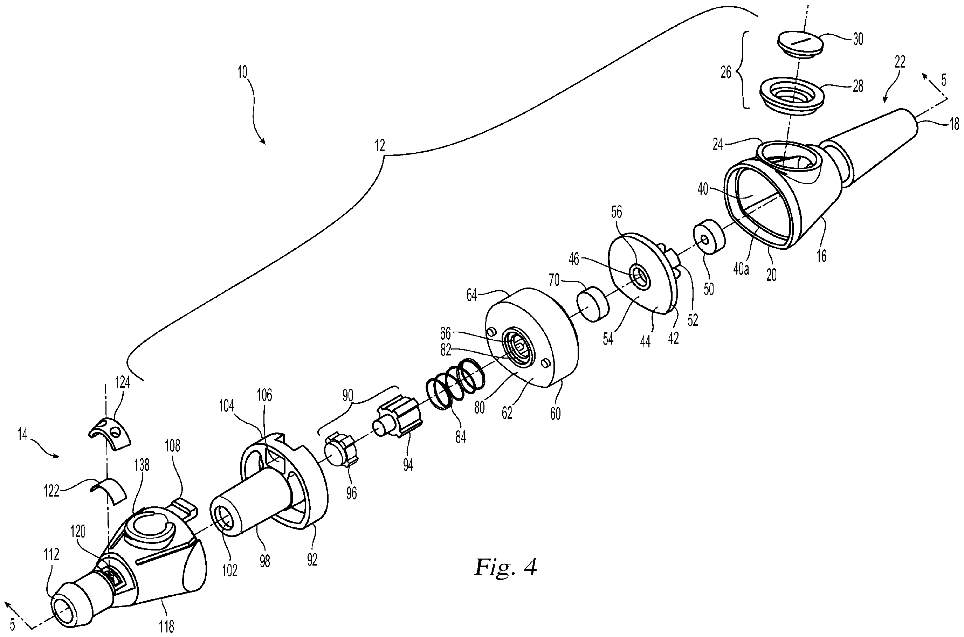

As best illustrated in Fig. 4, the catheter connector **16** also has an opening **24** that functions as a sampling port. The opening **24** preferably has a needleless port **26.** The needleless port **26** allows for insertion of a needleless syringe (not shown) to withdraw a sample of the liquid (generally urine) for testing. The needleless port **26** is preferably a resealable opening. The needleless port **26** has a main body **28** and the resealable portion **30,** the resealable portion **30** may or may not have a pre-cut slit therein. It is also possible that the needleless port **26** is a single unit and not made of two different portions. The main body **28** is preferably made of polypropylene and the resealable portion **30** is preferably made of a thermoplastic elastomer, but any appropriate materials may be used.

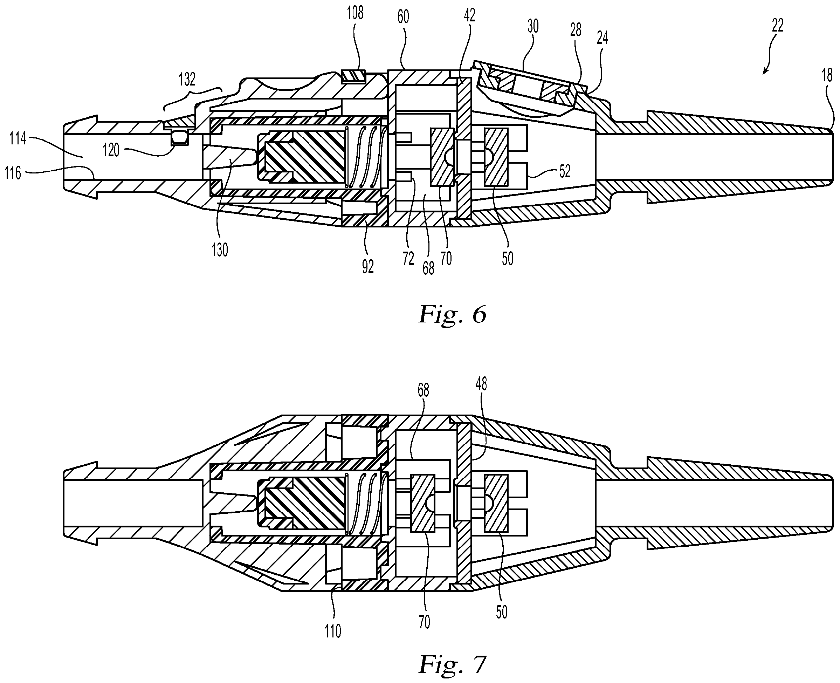

The catheter connector **16** also has an opening **40** at the distal end **20** that is in fluid communication with the inlet **22.** The opening **40** has a recessed portion **40a** adjacent the distal end **20** into which a first magnet housing **42** is disposed, sealing the opening **40** of the catheter connector **16.** See, e.g., Figs. 6-9. The first magnet housing **42** has a base plate **44** that has the same configuration as the opening **40** and an opening **46** in the base plate **44** to allow the liquid to pass therethrough. On a first side **48** of the base plate **44** a first magnet **50** is secured. The first magnet **50** is secured in extensions **52** that extend from the first side **48** of the base plate **44** to keep the first magnet **50** at a predetermined distance from the opening **46** and a second magnet, which is described in more detail below. The first magnet housing **42** preferably has on a second side **54** a raised portion **56** around the opening **46.** As described in more detail below, the raised portion provides a surface against which the second magnet can maintain contact to seal the opening **46.**

As best illustrated in Figs. 4 and 6-9, second magnet housing **60,** also a part of the first housing **12,** is attached to the first magnet housing **42** and the catheter connector **16** and extends distally from the catheter connector **16.** The second magnet housing **60** is preferably generally cup-shaped, having a base member **62** and a peripheral wall **64,** with an opening **66** in the base member **62** to allow the liquid to flow therethrough. Extending from the base member **62** toward the catheter connector **16** and the first magnet housing **40** are extensions **68** that slidingly hold a the second magnet **70.** Preferably, there are four extensions **68,** but there may be other numbers of extensions and still be within the scope of the invention. The extensions **68** also have a surface **72** to engage the second magnet **70** and prevent the second magnet **70** from moving too far distally (toward the base member **62**). The second magnet **70** is drawn magnetically toward the first magnet **50** causing the second magnet **70** to engage the raised portion **56** around the opening **46,** thereby closing the opening **46** and preventing the flow of liquid through the valve **10.** However, when sufficient liquid is present in the opening **40** and exerts pressure on the second magnet **70** sufficient to overcome the magnetic attraction between the two magnets **50,70,** then the second magnet **70** moves axially away from the opening **46** within the extensions **68** (but no farther than the surfaces **72**) to allow the liquid to drain through the opening **46** (and the tubing that is inserted into bladder of a patient). When the liquid has drained away and removes this force, then the magnetic attraction causes the second magnet **70** to once again close the opening **46.**

The second magnet housing **60** has on a bottom side **80** a recessed portion **82** to receive an elastic member **84** that engages and biases a sealing member **90** toward the second housing **14** in a drain end **92.** While a coil spring is illustrated as the elastic member **84,** any appropriate style of spring or elastic member may be used to bias the sealing member **90.** The sealing member **90** preferably has two elements, a main sealing member **94** and a resilient cover member **96** that is attached to the main sealing member **94.** However, the sealing member **90** may be one integral element rather than two separate elements and may be made from a single material rather than multiple materials.

The drain end **92,** which constitutes the last element of the first housing **12,** is attached to the second magnet housing **60** and is in fluid communication with the inlet **22** in the catheter connector **16.** See Figs. 4 and 6-9. The drain end **92** has a generally cylindrical center portion **98** in which the sealing member **90** is slidingly movable. The cylindrical center portion **98** has an inlet **100** and an outlet **102.** The sealing member **90** is, as noted above, biased away from the second magnet housing **60** and toward the outlet **102** of the drain end **92.** The drain end **92** also has an outer portion **104** that is secured to the bottom side **80** of the second magnet housing **60.** Between the cylindrical center portion **98** and the outer portion **104** is an opening **106** into which a cantilevered latch **108** from the second housing **14** is inserted to hold the second housing **14** to the first housing **12.**

The second housing **14** has a proximal end **110** and a distal end **112.** The second housing **14** has an inner opening **114** that extends between the proximal end **110** and the distal end **112** and defines an inner surface **116.** The second housing **14** also has a outside surface **118** and an opening **120** that extends between the outside surface **118** and the inner surface **116.** The opening **120** functions as a vent to allow air to enter the valve **10** and the liquid to move through the valve **10** and into the collection bag **140.** See Fig. 10. The opening **120** is preferably covered by a Tyvek covering **122** and a cover **124,** which allows air to enter into the valve **10** for complete (or near-complete) emptying of the valve **10** without allowing the liquid to escape therethrough.

Positioned within the inner opening **114** is a projection **130** that extends from central portion **132** of the inner opening **114** toward the proximal end **110.** The inner opening **114** also has in the central portion **132** (and preferably at the location of the distal end **134** of the projection **130**) a proximally-facing surface **136.** The inner opening **114** is configured and sized to receive the cylindrical center portion **98** of the first housing **12** therein and the proximally-facing surface **136** is positioned and configured such that the outer portions of outlet **102** of the drain end **92** make contact with the surface **136** and seal the valve **10** to prevent leaking when the two housings **12,14** are attached. The projection **130** is configured and sized, when the second housing **14** is connected to the first housing **12,** to engagethe sealing member **90** through the outlet **102** of the drain end **92** and move it axially and in a proximate direction (toward the inlet **22).** This allows for the liquid to drain from the first housing **12** in to the second housing **14.** Openings spaced around the bottom of the projection **130** allow the liquid to pass around the projection **130** and along the inner surface **114.** Since the proximal end **110** of the second housing **14** is, by way of the latch **108,** removably mounted to the first housing **12,** removing the second housing **14** also removes the projection **130** from the drain end **92,** allowing the sealing member **90** to seal the outlet **102,** even if the second magnet **70** moves to allow the liquid to pass through the opening **46.** This allows the patient to, at least temporarily, be separated from a collection bag **140,** which is illustrated in Fig. **10****.**

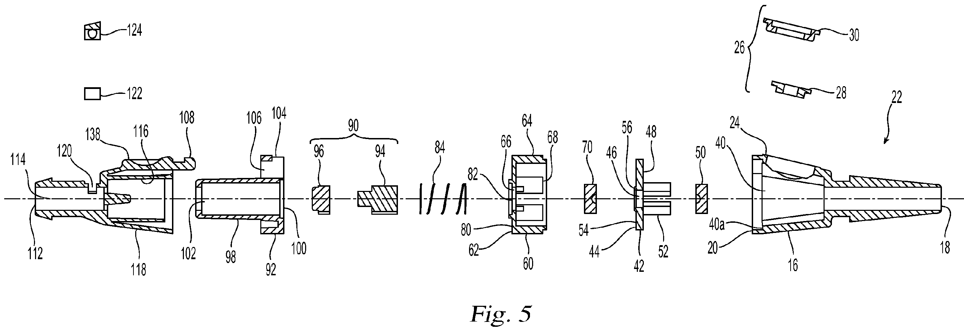

As noted above, the first and second housings **12,14** are removably attached to one another by the latch **108.** By "removably attached," Applicant means that the two housings **12,14** are intended to and can repeatedly engage and disengage one another without any other elements (e.g., glues, adhesives, bands, etc.), structures, or destroying any portions or parts that are intended to be used to attach housings **12,14.** As best seen in Figs. 5 and 6, the latch **108** is integral with the button **138,** which when pressed then causes the latch **108** to be disengaged from the outer portion **104** of the drain end **92,** and the housing **14** can be removed from housing **12.** It should also be noted that due to the tight fit of the cylindrical center portion **98** in the inner opening **114,** only one latch **108** needs to be used to maintain the connection between the two housings **12,14.** Other types of latches and numbers of latches may also be used with the valve **10** and still come within the scope of the present invention.

Turning now to Figs. 6-10, the operation of the valve **10** will be described in more detail. Fig. 6 is a cross sectional view of the valve **10** through the latch **108.** The position of the second magnet **70** is in the proximal position, that is the second magnet **70** is sealing off the opening **46** as it makes contact with the raised portion **56** around the opening **46.** As can be seen in the left side of the figure, the projection **130** has engaged the sealing member **90** through the outlet **102** since the second housing **14** is attached to the first housing **12.**

Fig. 7 is a cross sectional view of the valve **10** at a 90° angle to the view in Fig. 6. In this figure, the second magnet **70** is illustrated as if the liquid has exerted a sufficient force on the second magnet **70** to move it away from the raised portion **56** around the opening **46** and toward the surfaces **72.**

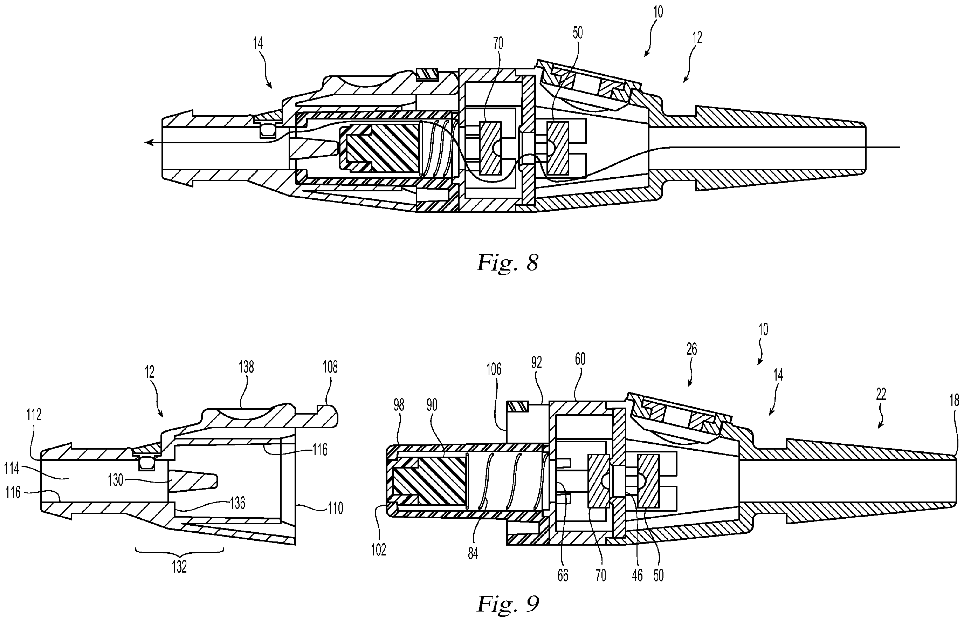

Fig. 8 is a cross sectional view of valve **10** in the same orientation as Fig. 6, but the second magnet **70** is is the open position and not closed as in Fig. 6. Additionally, the arrow indicates at least one path for the liquid to pass through the valve **10.**

Fig. 9 is a cross section of the valve **10** with the first housing **12** and the second housing **14** separated from one another. In this figure, it is clear that the projection **130** no longer engages the sealing member **90,** and the sealing member **90** is firmly in the outlet **102,** preventing the liquid from exiting the first housing **12.** This configuration allows a patient to disconnect the housings **12,14** for better mobility (or other reasons), and not have to worry about the liquid draining onto the floor or other equally distasteful places. While the second magnet **70** is illustrated as being against the raised portion **56** around the opening **46,** thereby closing the opening **46,** even if opening **46** were open, the liquid still would not leak from the first housing **12.**

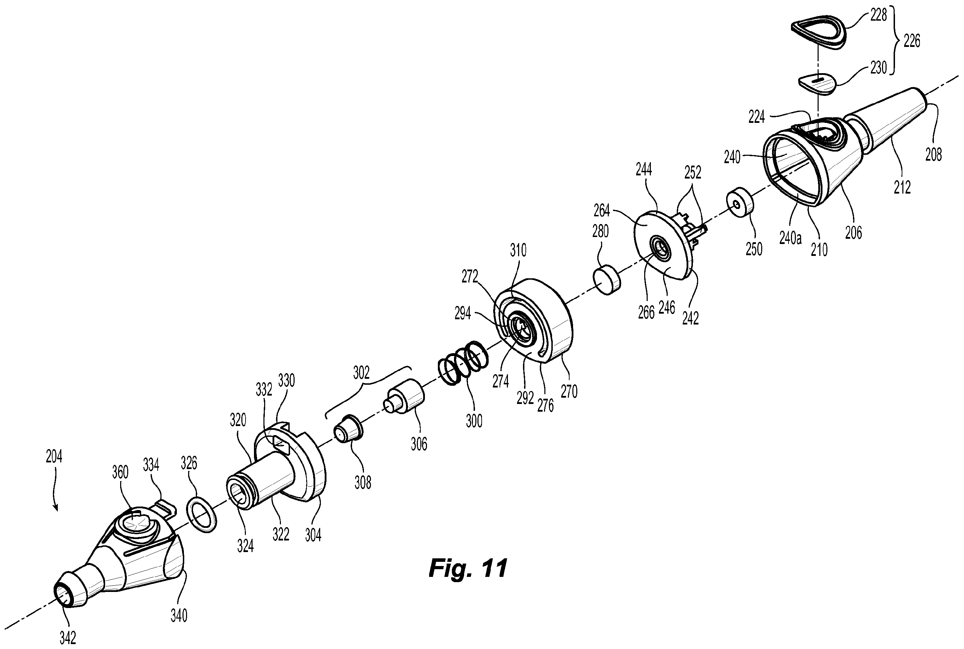

Another embodiment of a valve **200** according to the present invention is illustrated in Figs. 11-18. The valve **200** has a first housing **202** and a second housing **204** that is removably attachable to the first housing **202.** The first housing **202** has a catheter connector **206** having a proximal end **208** and a distal end **210,** the proximal end **208** of the catheter connector **206** has an inlet **212** that is configured to engage a tubing (see, e.g., Fig. 10). The inlet **212,** as illustrated, has a generally smooth outer surface that increases in diameter from the end of the inlet **212** toward the remaining portion of the catheter connector **206.** The inlet **212** may have any other configuration that allows for connection to tubing and still fall within the scope of the present invention. The first and second housings **202,204** are preferably made from a polycarbonate.

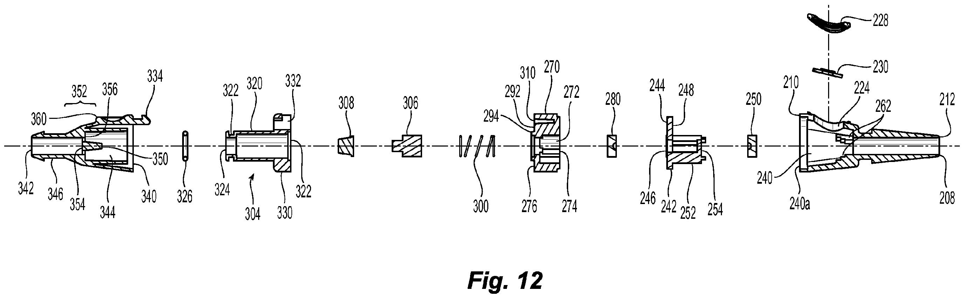

As best illustrated in Figs. 11 and 12, the catheter connector **206** also has an opening **224** that functions as a sampling port. The opening **224** preferably has a needleless port **226.** The needleless port **226** allows for insertion of a needleless syringe (not shown) to withdraw a sample of the liquid (generally urine) for testing. The needleless port **226** is preferably a resealable opening. The needleless port **226** has a main body **228** and the resealable portion **230,** the resealable portion **230** may or may not have a pre-cut slit therein. It is also possible that the needleless port **226** is a single unit and not made of two different portions. The main body **228** is preferably made of polypropylene and the resealable portion **230** is preferably made of Dynaflex® thermoplastic elastomer (available from GLS Corp.), but any appropriate materials may be used. Additionally, a syringe with a needle may also be used with the needleless port **226,** as discussed in more detail below regarding the configuration of the needleless port **226** to the internal structures.

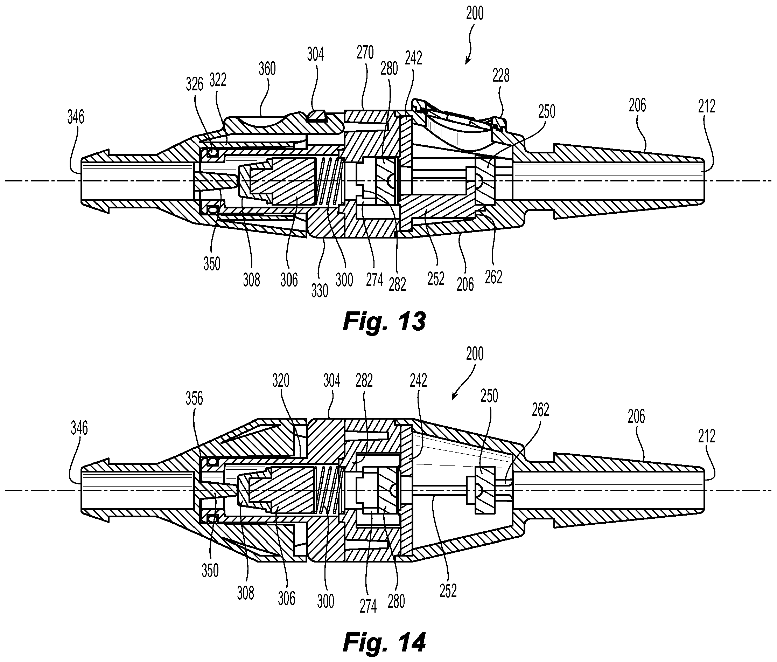

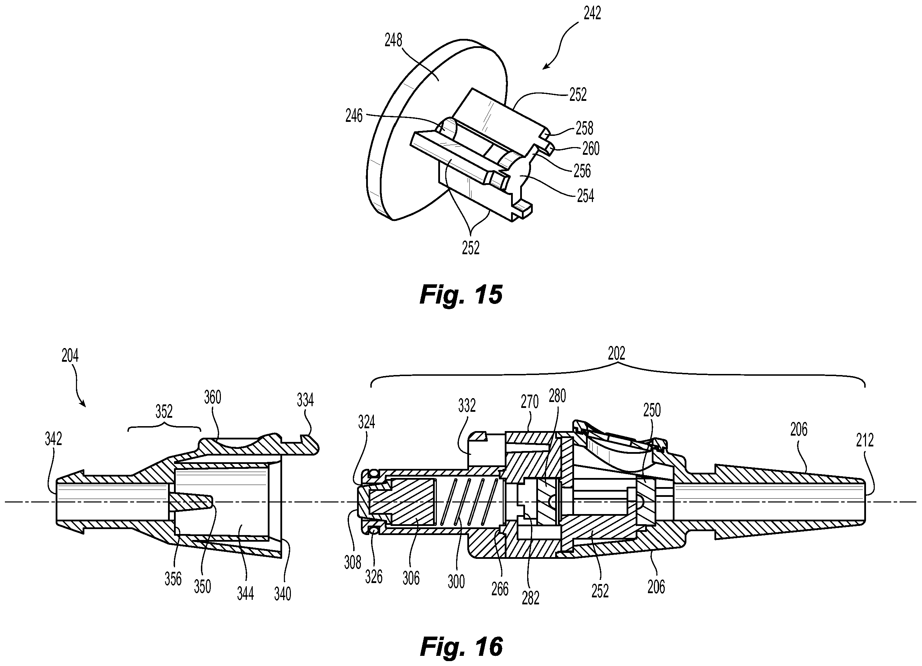

The catheter connector **206** also has an opening **240** at the distal end **220** that is in fluid communication with the inlet **212.** The opening **240** has a recessed portion **240a** adjacent the distal end **210** into which a first magnet housing **242** is disposed, sealing the opening **240** of the catheter connector **206.** See, e.g., Figs. 13 and 14. The first magnet housing **242** is preferably a monolithic element that is injection molded as a single element from Dynaflex® thermoplastic elastomer. However, the various parts of the first magnetic housing **242** may also be assembled and attached to one another by appropriate means for the materials (e.g., adhesives, ultrasonic welding, etc.). As best seen in Fig. 15, first magnetic housing **242** has a base plate **244** that has the same configuration as the opening **240** and an opening **246** in the base plate **244** to allow the liquid to pass therethrough. On a first side **248** of the base plate **244** a first magnet **250** is secured. The first magnet **250** is secured in between extensions **252** and on a support member **254.** The extensions **252** extend from the first side **248** of the base plate **244,** and in conjunction with the support member **254,** keep the first magnet **250** in a predetermined relationship with the opening **246** and a second magnet, which is described in more detail below. The support member **254** also provides support and stability to the extensions **252.** The top of the extensions **252** have a stepped configuration that serves two purposes. First, the extensions **252** have an inner step **256** that preferably engages the first magnet **250** to support and contain the first magnet **250** relative to the inlet **212** and the opening **240.** The second purpose of the outer step **258** and the top surface **260** of the extensions **252** is to engage the inner surface **262** of the catheter connector **206.** As best illustrated in Figs. 11, 13 and 18, the catheter connector **206** has a similar stepped configuration **262** that aligns with and engages the steps **256,258.** When the first magnet housing **242** is inserted into the catheter connector **206** with the magnet **250** attached thereto, the steps **256,258** engage and may even compress slightly as the valve **200** is assembled.

The first magnet housing **242** also preferably has on a second side **264** a raised portion **266** around the opening **246.** As described in more detail below, the raised portion **266** provides a surface against which the second magnet can maintain contact to seal the opening **246.**

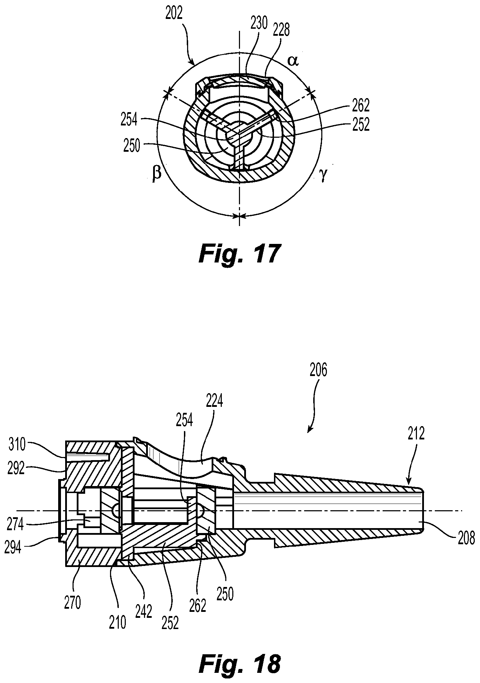

As illustrated in Fig. 17, the extensions **252** are preferably disposed evenly around the opening **246** in the first magnet housing **242.** As illustrated, the extensions **252** have an angle between them, including **α**, **β**, and **γ**. In Fig. 17, the angles are all the same (**α=β=γ)**, but they could have different values. Additionally, a line **A** that is orthogonal to the valve **200** and passes through the center of the valve **200** bisects the opening **224** and needleless port **226.** Line A also preferably bisects the angle formed by two adjacent extensions **252** that are disposed closest to the opening **224.** In this way, if the user or a healthcare worker were to use a syringe with a needle to take a sample, the needle would have a substantial amount of space in which to retrieve the sample and not be inserted into the extensions.

As best illustrated in Figs. 11, 13, and 14, second magnet housing **270,** also a part of the first housing **202,** is attached to the first magnet housing **242** and the catheter connector **206** and extends distally from the catheter connector **206.** The second magnet housing **270** is preferably generally cup-shaped with a central opening **272** to allow the liquid to flow therethrough. The central opening **272** has extensions **274** that extend from the distal end **276** toward the catheter connector **206** and the first magnet housing **240** that slidingly hold a the second magnet **280.** Preferably, there are three extensions **274,** but there may be other numbers of extensions and still be within the scope of the invention. The extensions **274** also have a surface **282** to engage the second magnet **280** and prevent the second magnet **280** from moving too far distally (toward the distal end **276**). The second magnet **280** is drawn magnetically toward the first magnet **250** causing the second magnet **280** to engage the raised portion **266** around the opening **246,** thereby closing the opening **246** and preventing the flow of liquid through the valve **200.** However, when sufficient liquid is present in the opening **240** and exerts pressure on the second magnet **280** sufficient to overcome the magnetic attraction between the two magnets **250,280,** then the second magnet **280** moves (is pushed) axially away from the opening **246** within the extensions **274** (but no farther than the surfaces **282**) to allow the liquid to drain through the opening **246** (and the tubing that is inserted into bladder of a patient). When the liquid has drained away and removes this force, then the magnetic attraction causes the second magnet **280** to once again close the opening **246.**

The second magnet housing **270** has on a bottom side **292** an elevated ring portion **294** that engages and centrally maintains an elastic member **300,** which in turn, engages and biases a sealing member **302** toward the second housing **204** in a drain end **304.** While a coil spring is illustrated as the elastic member **300,** any appropriate style of spring or elastic member may be used to bias the sealing member **302.** The sealing member **302** preferably has two elements, a main sealing member **306** and a resilient cover member **308** that is attached to the main sealing member **302.** However, the sealing member **302** may be one integral element rather than two separate elements and may be made from a single material rather than multiple materials. The second magnet housing **270** also has a recessed portion **310.** The recessed portion **310,** in this embodiment, is simply to reduce the volume and thereby the weight of the second magnet housing **270** as it is machined from a single block of material. The recessed portion **310** plays no other role in the valve **200.**

The drain end **304,** which constitutes the last element of the first housing **202,** is attached to the second magnet housing **270** and is in fluid communication with the inlet **212** in the catheter connector **206.** See Figs. 13-14 and 16. The drain end **304** has a generally cylindrical center portion **320** in which the sealing member **302 is** slidingly movable. The cylindrical center portion **320** has an inlet **322** and an outlet **324.** The sealing member **302** is, as noted above, biased away from the second magnet housing **270** and toward the outlet **324** of the drain end **304.** The drain end **304** also has an outer portion **330** that is secured to the bottom side **292** of the second magnet housing **270.** Between the cylindrical center portion **320** and the outer portion **330** is an opening **332** into which a cantilevered latch **334** from the second housing **204** is inserted to hold the second housing **204** to the first housing **202.**

The cylindrical center portion **320** has a groove **322** near the outlet **324** and preferably adjacent the outlet **324.** The location of the groove **324** need only be such that an elastic element **326,** such as an o-ring, can engage the second housing **204** as described in more detail below.

The second housing **204** has a proximal end **340** and a distal end **342.** The second housing **204** has an inner opening **344** that extends between the proximal end **340** and the distal end **342** and defines an inner surface **346.**

Positioned within the inner opening **344** is a projection **350** that extends from central portion **352** of the inner opening **344** toward the proximal end **340.** The inner opening **344** also has in the central portion **352** (and preferably at the location of the distal end **354** of the projection **350**) a proximally-facing surface **356.** The inner opening **344** is configured and sized to receive the cylindrical center portion **320** of the first housing **202** therein and the proximally-facing surface **356** is positioned and configured such that the outer portions of outlet **324** of the drain end **304** make contact with the proximally-facing surface **356** (and in particular the elastic element **326** in the groove **322**) and seal the valve **200** to prevent leaking when the two housings **202,204** are attached. The projection **350** is configured and sized, when the second housing **204** is connected to the first housing **202,** to engage the sealing member **302** through the outlet **324** of the drain end **304** and move it axially and in a proximate direction (toward the inlet **212**). This allows for the liquid to drain from the first housing **202** in to the second housing **204.** Openings spaced around the bottom of the projection **350** allow the liquid to pass around the projection **350** and along the inner surface **364.** Since the proximal end **340** of the second housing **204** is, by way of the latch **334,** removably mounted to the first housing **202,** removing the second housing **204** also removes the projection **350** from the drain end **304,** allowing the sealing member **302** to seal the outlet **324,** even if the second magnet **280** moves to allow the liquid to pass through the opening **246.** This allows the patient to, at least temporarily, be separated from a collection bag, as illustrated in Fig. **10****.**

As noted above, the first and second housings **202,204** are removably attached to one another by the latch **334** as noted above. By "removably attached," Applicant means that the two housings **202,204** are intended to and can repeatedly engage and disengage one another without any other elements (e.g., glues, adhesives, bands, etc.), structures, or destroying any portions or parts that are intended to be used to attach housings **202,204.** As best seen in Figs. 13 and 14, the latch **334** is integral with the button **360,** which when pressed then causes the latch **334** to be disengaged from the outer portion **330** of the drain end **304,** and the housing **204** can be removed from housing **202.** It should also be noted that due to the tight fit of the cylindrical center portion **320** in the inner opening **344,** only one latch **334** needs to be used to maintain the connection between the two housings **202,204.** Other types of latches and numbers of latches may also be used with the valve **200** and still come within the scope of the present invention.

It will be apparent to those skilled in the art that various modifications and variations can be made to the present invention without departing from the scope of the invention. Thus it is intended that the present invention cover the modifications and variations of this invention provided they come within the scope of the appended claims and their equivalents.

## Claims

1. A valve for regulating the flow of a liquid therethrough comprising:
a first housing (12, 202) having an inlet and an outlet, the first housing (12, 202) having a first magnet housing (42, 242) disposed therein, the first magnet housing (42, 242) having a base (44, 244), the base (44, 244) having a first side and a second side and at least two extensions (52, 252) extending from the first side;
a first magnet (50, 250) retained by the at least two extensions (52, 252) between the first magnet housing (42, 242) and the first housing (12, 202);
a second magnet (70, 280), the second magnet (70, 280) movable relative to the first magnet (50, 250); and
a second housing (14, 204), the second housing (14, 204) having an opening extending therethrough;
whereby
the second magnet (70, 280) is disposed in the first housing (12, 202);
the second housing (14, 204) is removably attachable to the first housing (12, 202);
a sealing member (90, 302) disposed in the first housing (12, 202) adjacent the outlet, the sealing member (90, 302) sealing the outlet in the first housing (12, 202) when the second housing (14, 204) is disengaged from the first housing (12, 202),
wherein the sealing member (90, 302) includes an elastic member (84, 300) that engages a portion of the first housing (12, 202), and wherein the second housing (70, 280) has a projection (130, 350) that engages the sealing member (90, 302) and compresses the elastic member (84, 300) when the second housing (70, 280) engages the first housing (12, 202) to move the sealing member (90, 302) toward the inlet in the first housing (12, 202) and opens the outlet.

2. The valve according to claim 1, wherein the first housing (12, 202) has an outer surface, the outer surface having a groove adjacent the outlet and the elastic member disposed within the groove, the elastic member to engage an inner surface of the opening of the second housing (14, 202) when attached thereto.

3. The valve according to claim 1, wherein the first magnet housing (42, 242) is comprised of flexible material.

4. The valve according to claim 1, wherein the first magnet housing (42, 242) has an opening in the base and a ridge surrounding the opening on the second side.

5. The valve according to claim 1, wherein the first magnet housing (42, 242) is a monolithic element.

6. The valve according to claim 4, further comprising a sampling port disposed in the first housing (12, 202) and wherein the at least two extensions (52, 252) are disposed around the opening in the base and have an angle between them, the angle being 180 degrees or less, the at least two extensions (52, 252) being disposed in the first housing (12, 202) such the sampling port bisects the angle between the at least two extensions when the first magnet housing (42, 242) is disposed within the first housing.

7. The valve according to claim 1, wherein the outlet of the first housing (12, 202) engages the opening in the second housing (14, 204).

8. The valve according to claim 1, wherein the opening of the second housing (14, 204) has the projection (130, 350) that engages the sealing member (90, 302) when the second housing (14, 204) engages the first housing (12, 202), the projection (130, 350) extending from a base member, the base member extending across at least a portion of the opening in the second housing (12, 202).

9. The valve according to claim 8, wherein the base member extends across the opening, the base member having at least one opening therein to allow liquid to flow through the second housing and around the projection (130, 350).

10. The valve according to claim 1, further comprising a first latch member (108, 334) disposed on the first housing (12, 202) and a second latch member (108, 334) disposed on the second housing (14, 214), the two latch members configured to engage each other.

## Patentansprüche

1. Ventil zur Regulierung des Durchflusses einer Flüssigkeit, umfassend:
ein erstes Gehäuse (12, 202) mit einem Einlass und einem Auslass, wobei das erste Gehäuse (12, 202) ein erstes darin angeordnetes Magnetgehäuse (42, 242) aufweist, wobei das erste Magnetgehäuse (42, 242) eine Basis (44, 244) aufweist, wobei die Basis (44, 244) eine erste Seite und eine zweite Seite und mindestens zwei Fortsätze (52, 252) aufweist, die sich von der ersten Seite erstrecken;
einen ersten Magneten (50, 250), der durch die mindestens zwei Fortsätze (52, 252) zwischen dem ersten Magnetgehäuse (42, 242) und dem ersten Gehäuse (12, 202) gehalten wird;
einen zweiten Magneten (70, 280), wobei der zweite Magnet (70, 280) relativ zu dem ersten Magneten (50, 250) beweglich ist; und
ein zweites Gehäuse (14, 204), wobei das zweite Gehäuse (14, 204) eine Öffnung aufweist, die sich dadurch erstreckt;
wobei
der zweite Magnet (70, 280) in dem ersten Gehäuse (12, 202) angeordnet ist;
das zweite Gehäuse (14, 204) entfernbar an dem ersten Gehäuse (12, 202) befestigt ist;
ein Dichtungselement (90, 302), das in dem ersten Gehäuse (12, 202) neben dem Auslass angeordnet ist, wobei das Dichtungselement (90, 302) den Auslass in dem ersten Gehäuse (12, 202) abdichtet, wenn das zweite Gehäuse (14, 204) nicht in Eingriff mit dem ersten Gehäuse (12, 202) steht,
wobei das Dichtungselement (90, 302) ein elastisches Element (84, 300) umfasst, das mit einem Abschnitt des ersten Gehäuses (12, 202) in Eingriff steht, und wobei das zweite Gehäuse (70, 280) einen Vorsprung (130, 350) aufweist, der mit dem Dichtungselement (90, 302) in Eingriff steht und das elastische Element (84, 300) komprimiert, wenn das zweite Gehäuse (70, 280) mit dem ersten Gehäuse (12, 202) in Eingriff steht, um das Dichtungselement (90, 302) zu dem Einlass in dem ersten Gehäuse (12, 202) hin zu bewegen, und den Auslass öffnet.

2. Ventil nach Anspruch 1, wobei das erste Gehäuse (12, 202) eine Außenfläche aufweist, wobei die Außenfläche eine Nut neben dem Auslass aufweist und das elastische Element in der Nut angeordnet ist, wobei das elastische Element mit einer Innenfläche der Öffnung des zweiten Gehäuses (14, 202) in Eingriff steht, wenn es daran befestigt wird.

3. Ventil nach Anspruch 1, wobei das erste Magnetgehäuse (42, 242) aus flexiblem Material besteht.

4. Ventil nach Anspruch 1, wobei das erste Magnetgehäuse (42, 242) eine Öffnung in der Basis und eine Erhöhung, die die Öffnung auf der zweiten Seite umgibt, aufweist.

5. Ventil nach Anspruch 1, wobei das erste Magnetgehäuse (42, 242) ein monolithisches Element ist.

6. Ventil nach Anspruch 4, ferner umfassend einen Probenahmeanschluss, der in dem ersten Gehäuse (12, 202) angeordnet ist, und wobei die mindestens zwei Fortsätze (52, 252) um die Öffnung in der Basis herum angeordnet sind und einen Winkel zwischen ihnen aufweisen, wobei der Winkel 180 Grad oder weniger beträgt, die mindestens zwei Fortsätze (52, 252) in dem ersten Gehäuse (12, 202) derart angeordnet sind, dass der Probenahmeanschluss den Winkel zwischen den mindestens zwei Fortsätzen halbiert, wenn das erste Magnetgehäuse (42, 242) in dem ersten Gehäuse angeordnet ist.

7. Ventil nach Anspruch 1, wobei der Auslass des ersten Gehäuses (12, 202) mit der Öffnung in dem zweiten Gehäuse (14, 204) in Eingriff steht.

8. Ventil nach Anspruch 1, wobei die Öffnung des zweiten Gehäuses (14, 204) den Vorsprung (130, 350) aufweist, der mit dem Dichtungselement (90, 302) in Eingriff steht, wenn das zweite Gehäuse (14, 204) mit dem ersten Gehäuse (12, 202) in Eingriff steht, wobei sich der Vorsprung (130, 350) von einem Basiselement erstreckt, wobei sich das Basiselement über mindestens einen Abschnitt der Öffnung in dem zweiten Gehäuse (12, 202) erstreckt.

9. Ventil nach Anspruch 8, wobei das Basiselement sich quer über die Öffnung erstreckt, wobei das Basiselement mindestens eine Öffnung darin aufweist, damit Flüssigkeit durch das zweite Gehäuse und um den Vorsprung (130, 350) herum fließen kann.

10. Ventil nach Anspruch 1, ferner umfassend ein erstes Verriegelungselement (108, 334), das auf dem ersten Gehäuse (12, 202) angeordnet ist, und ein zweites Verriegelungselement (108, 334), das auf dem zweiten Gehäuse (14, 214) angeordnet ist, wobei die beiden Verriegelungselemente so ausgestaltet sind, dass sie miteinander in Eingriff stehen.

## Revendications

1. Robinet pour réguler l'écoulement d'un liquide le traversant, comprenant :
un premier boîtier (12, 202) comportant une entrée et une sortie, le premier boîtier (12, 202) comportant un premier boîtier (42, 242) d'aimant disposé à l'intérieur, le premier boîtier (42, 242) d'aimant comportant une base (44, 244), la base (44, 244) comportant un premier côté et un second côté et au moins deux extensions (52, 252) s'étendant depuis le premier côté ;
un premier aimant (50, 250) retenu par les au moins deux extensions (52, 252) entre le premier boîtier (42, 242) d'aimant et le premier boîtier (12, 202) ;
un second aimant (70, 280), le second aimant (70, 280) étant mobile par rapport au premier aimant (50, 250) ; et
un second boîtier (14, 204), le second boîtier (14, 204) comportant une ouverture le traversant,
grâce auquel :
le second aimant (70, 280) est disposé dans le premier boîtier (12, 202) ;
le second boîtier (14, 204) peut être fixé de manière amovible au premier boîtier (12, 202) ;
un élément d'étanchéité (90, 302) disposé dans le premier boîtier (12, 202) tout à côté de la sortie, l'élément d'étanchéité (90, 302) fermant hermétiquement la sortie dans le premier boîtier (12, 202) quand le second boîtier (14, 204) est désaccouplé du premier boîtier (12, 202),
dans lequel l'élément d'étanchéité (90, 302) comprend un élément élastique (84, 300) qui prend appui sur une partie du premier boîtier (12, 202), et dans lequel le second boîtier (70, 280) comporte une avancée (130, 350) qui prend appui sur l'élément d'étanchéité (90, 302) et comprime l'élément élastique (84, 300) quand le second boîtier (70, 280) entre en prise avec le premier boîtier (12, 202) pour déplacer l'élément d'étanchéité (90, 302) vers l'entrée dans le premier boîtier (12, 202) et ouvre la sortie.

2. Robinet selon la revendication 1, dans lequel le premier boîtier (12, 202) comporte une surface extérieure, la surface extérieure comportant une rainure adjacente à la sortie et l'élément élastique disposé à l'intérieur de la rainure, l'élément élastique étant configuré pour entrer en prise avec une surface intérieure du second boîtier (14, 202) quand il est fixé à celui-ci.

3. Robinet selon la revendication 1, dans lequel le premier boîtier (42, 242) d'aimant est constitué d'une matière souple.

4. Robinet selon la revendication 1, dans lequel le premier boîtier (42, 242) d'aimant comporte une ouverture dans la base et une crête entourant l'ouverture sur le second côté.

5. Robinet selon la revendication 1, dans lequel le premier boîtier (42, 242) d'aimant est un élément monolithique.

6. Robinet selon la revendication 4, comprenant en outre un point de prélèvement dans le premier boîtier (12, 202) et dans lequel les au moins deux extensions (52, 252) sont disposées autour de l'ouverture dans la base et font entre elles un angle, l'angle étant inférieur ou égal à 180 degrés, les au moins deux extensions (52, 252) étant disposées dans le premier boîtier (12, 202) de telle sorte que le point de prélèvement bissecte l'angle entre les au moins deux extensions quand le premier boîtier (42, 242) d'aimant est disposé à l'intérieur du premier boîtier.

7. Robinet selon la revendication 1, dans lequel la sortie du premier boîtier (12, 202) entrer en prise avec l'ouverture dans le second boîtier (14, 204).

8. Robinet selon la revendication 1, dans lequel l'ouverture du second boîtier (14, 204) comporte l'avancée (130, 350) qui entre en prise avec l'élément d'étanchéité (90, 302) quand le second boîtier (14, 204) entre en prise avec le premier boîtier (12, 202), l'avancée (130, 350) s'étendant depuis un élément de base, l'élément de base s'étendant d'un côté à l'autre d'au moins une partie de l'ouverture dans le second boîtier (12, 202).

9. Robinet selon la revendication 8, dans lequel l'élément de base s'étend d'un côté à l'autre de l'ouverture, l'élément de base comportant en lui au moins une ouverture pour permettre à un liquide de s'écouler à travers le second boîtier et autour de l'avancée (130, 350).

10. Robinet selon la revendication 1, comprenant en outre un premier élément de verrouillage (108, 334) disposé sur le premier boîtier (12, 202) et un second élément de verrouillage (108, 334) disposé sur le second boîtier (14, 214), les deux éléments de verrouillage étant configurés pour entrer en prise l'un avec l'autre.
